# EUROPEAN PATENT SPECIFICATION

(11) **EP 2 694 202 B1**
(45) Date of publication and mention of the grant of the patent: **06.05.2015**
(21) Application number: 12727452.0
(22) Date of filing: 02.03.2012
(51) Int. Cl.: B01J 20/20, C01B 31/02, A61K 33/44, B01J 20/30

(54) **METHOD FOR PRODUCTION OF "INGO-2" CARBON ENTEROSORBENT**
VERFAHREN ZUR HERSTELLUNG VON INGO-2-KOHLENSTOFFENTEROSORBENT
PROCÉDÉ POUR LA PRODUCTION D'UN ENTÉROSORBANT CARBONÉ « INGO-2 »

(30) Priority: 08.04.2011 KZ 20110359
(43) Date of publication of application: 12.02.2014
(73) Proprietor: Republican State Enterprise, Based On The Right Of Economic Management, "Al-Farabi Kazakh National University", Almaty 050040 (KZ); Republican State Enterprise, Based On The Right Of Economic Management, "Institute of Combustion Problems", Almaty (KZ); "NPTC "Jalyn" Limited Liability Company, Almaty (KZ)
(72) Inventor: MANSUROV, Zulhair Aymuhametovich, Almaty 050057 (KZ); SAVICKAYA, Irina Stanislavovna, Almaty 050035 (KZ); KISTAUBAYEVA, Aida Serikovna, Almaty 050007 (KZ); BIYSENBAYEV, Mahmut Akhmetzhanovich, Almaty 050026 (KZ); TULEYBAYEVA, Shalpan Aliyevna, Almaty 050000 (KZ); NIKOLAYEVA, Antonina Fedorovna, Almaty 050012 (KZ)
(74) Representative: von Füner, Nicolai
(86) International application number: PCT/KZ2012/000002
(87) International publication number: WO 2012/138207

(56) References cited:
- EP-A1- 2 060 535
- US-A1- 2005 196 336

## Description

The present invention relates to the pharmaceutical industry. More specifically, the present invention relates to medical enterosorbents for peroral administration with acute poisoning and other endogenous and exogenous intoxications.

Currently, search for user-friendly materials having high sorption properties in relation to a broad spectrum of toxics and microorganisms is one of actual problems in enterosorption. Using enterosorbents as agents of sorption therapy can provide a solving the complex problem including detoxification of gastrointestinal tract and normalization of bowels status.

A carbon adsorbent of seeds of fruit-trees is known (patent RU No 2105714 published of 12.03.1996, IPC C01B31/08).

However the carbon adsorbent has a shortage included low adsorption capacity (absorbing ability) in relation to toxins having large and branched molecules (e.g., enterotoxin of gram-negative bacteria).

The closest solution on a technical essence and reached technical effect with respect to the invention is a method for production of a carbon enterosorbent from seeds of fruit-trees (patent RU No 2166990, published of 08.02.2000, IPC B01J20/20). The method for production of the carbon enterosorbent includes a carbonization of the seed raw material followed by an isothermal ageing at temperature of 750 ± 10°C for a time ranging from 20 to 30 minutes, activation, magnetic separation, processing adsorbent with a sharp steam at temperature ranging from 200 to 250°C and the coal and steam ratio equal to 1:7-10.3, washing and drying.

Shortage of the method are using the specific raw material being fruit seeds required processing by a special equipment, difficult production technology of the sorbent and low sorption activity of the obtained product in respect of microbial cells and enterotoxins.

Advantages of the invention are a simplification of a method, diversification of enterosorbents from plant raw materials, utilization of rice husks.

The advantages of the invention are reached by a method for production carbon enterosorbent, including a carbonization of a plant raw material followed by an isothermal ageing for a time ranging from 20 to 30 minutes, washing, drying, wherein rice husks are used as the plant raw material, the isothermal ageing is carried out at temperature of 650 ± 10°C, and before washing the carbonized plant material is demineralized with 6M solution of hydrochloric acid at 100°C for a time ranging from 4 to 10 hours followed by neutralization with alkali.

The additional techniques provided in the method according to the invention lead to substantial increasing quality of the target enterosorbent; in particular the enterosorbent adsorbability is essentially raised, including adsorption of microbic cells and enterotoxin. INGO-2 can be used as the enterosorbent reducing overgrowth of gram-negative bacteria in large intestine. Thus the enterosorbent according to the present invention does not have negative effect on bowels microflora.

Due to the developed porous structure, INGO-2 enterosorbent based on carbonized rice husk has a high specific surface and is capable to adsorb various organic and inorganic compounds, including microbic lipopolysaccharide (LPS). This provides occurrence of qualitatively new sorption properties. Due to new found properties the preparation can sorb high-molecular and medium-molecular compounds including, in particular, enterotoxins of a microbic origin. INGO-2 enterosorbent has high sorption ability associated not only with the developed internal surface but also with various functional groups in its macromolecules. This allows binding and peroral removing bacterial cells and waste products, exogenous toxins and endogenous toxins penetrated into gastrointestinal tract. INGO-2 enterosorbent restores biocenosis of bowels, reduces concentration of toxins in blood, plasma and ascitic fluid, and restores other biochemical indexes affected by exotoxemia and endotoxemia.

The present invention consists in the following. To produce a carbon enterosorbent with ratio of sum of mesopore and macropore volumes to micropore volume ranging from 1.2 to 1.4, mineral impurity ranging from 1% to 2%, aqueous extract pH ranging from 5.0 to 5.5 and mechanical strength ranging from 89% to 97%, rice husks are fed to a reactor-carbonator where the material is heated from 10°C to 650 ± 10°C, moving along a retort, then the material is moved in an isothermal ageing reactor heated to 650 ± 10°C along the full length. Remaining the carbonized rice husks at 650 ± 10°C for a time ranging from 20 to 30 minutes is reached by tilting angle of the reactor or changing rotary speed, and then so treated rice husks are fed to a stirred reactor, treated with 6M solution of hydrochloric acid two times and boiled with agitation for 6 hours. Processing is recycled with a new portion of the hydrochloric acid solution. Further the product is neutralized with 3% solution of caustic soda at heating and agitation up to pH ranging from 5.0 to 5.5, the product is separated from the solution and dried up to an air-dry condition, consequently the enterosorbent having sorptive capacity ranging from 1.8 to 2.8 mol/g is received.

The claimed carbonization temperature is found by experiments. The electron-microscopic analysis of the claimed sorbent showed that a carbon with the structure needed for achievement of the invention advantages is not formed at the carbonization temperature lower than 600°C, sorbent pores are clogged up by carbon-black at the carbonization temperature higher than 700°C, this also reduces the enterosorbent sorptive capacity and degree of sorption.

Thus, the claimed carbonization temperature of 650°C is necessary and sufficient for achievement of the advantages reached by the invention.

Active coals contain the mineral impurity identified as ashes in accordance with State Standard 12596, ranging from 2.9% to 20%. Mineral components consist of, by weight: silicon dioxide ranging from 40% to 60%; aluminum oxide ranging from 2% to 20%; ferric oxide ranging from 3% to 20%; calcium, potassium, magnesium, sodium oxides ranging from 0.5% to 5%; copper, nickel, manganese, chrome, cobalt oxides ranging from 0.01% to 0.2%. Ashes presence does not meet the requirements of use areas, such as the food industry and public health.

To prepare production of carbon sorbents from plant raw materials, having mineral components lower than from 0.2% to 0.5%, the process demineralization is carried out in accordance with general methods. Hydrochloric acid sufficiently full removes mineral components. Further, the sorbent neutralisation with caustic soda to the basic state and washing with the distilled water are carried out. Obtained data exhibites that only pH ranging from 5.5 to 8.0 provides high adsorption properties.

Thus, novelty of the invention consists in the different carbonization operation and using the new plant raw material in comparison with the prior art. The combination of properties of raw material provides necessary sorption characteristics which along with carbonization conditions and the subsequent processing are formed the developed porous structure of the sorbent granules, developed further during thermal processing of the material.

Test of the end product on sorption activity is carried out in accordance with State Standard technique based on adsorption activity with methylene blue, i.e. State Standard 4453 - 74. A specific surface is measured by argon thermal desorption method. LPS sorption is studied as follows: 1 g of a sterile carbonized material is added to 40 ml of LPS solution with concentration of 1 ng/ml and the obtained samples are stirred up on a shaker with a speed of 110 rpm at the room temperature (20 ± 0.7)°C. Then the solution samples for LPS concentration tests are taken. LPS concentration in the medium is detected by photometric test on microtablets with the detector set QCL-1000 Chromogenic LAL Endpoint Assay (Lonza Group Ltd, Switzerland) and photometric scanner (Bio-Rad Co., USA).

Optical density of the reaction mixture is measured at a wavelength of 405 nm on microtablets, using the photometric scanner (Bio-Rad Co., USA). To calculate LPS concentration a calibration curve ranging from 0.1 to 1 EU/ml (10-100 pkg/ml) is drown up.

Numbers of adsorbed microorganism cells are defined on difference between cell concentration of tested stains in the initial medium and cell concentration of tested stains in supernatant fraction by results of a seeding on agar mediums following an incubation with the sorbent.

Studying of sorbent effects on bowels microflora of animals are taken in experiments *in vivo* in 3 groups of white outbred rats, 10 units for each. The first control group is included intact animals; the second group is fed with the enterosorbent from rice husks (INGO-2); the third group is fed with the sorbent from carbon-bearing peach stones (CBPS).

The invention is illustrated by examples of the method for production and test examples of enterosorbent according to the invention.

### Example 1

50 g of rice husks (500 sm³) are poured into a form and put in a reactor. Carbonization is carried out without air access in a static mode at temperature rise up 10°C per a minute to 650°C by isothermal ageing for 30 minutes. Then the carbonized product is crushed in a powder form with a particle size less than 0.2 mm, or in a granule form with a particle size ranging from 0.2 to 1.0 mm. (mas.%). From the produced carbon sorbent a fraction with a particle size ranging from 0.2 to 0.6 mm is selected and put in a stirred reactor, then processed with 6 M solution of hydrochloric acid two times and boiled with stirring for 6 hours. Processing is recycled with a new portion of the hydrochloric acid solution. Further a product is neutralized with 3% solution of caustic soda to pH ranging from 5.0 to 5.5 at heating and stirring, the product is separated from the solution and dried up to an air-dry condition; as a result an enterosorbent with sorption capacity ranging from 1.8 to 2.8 mol/g is obtained.

### Example 2

Specific surface, porosity and adsorption activity of INGO-2 and CBPS carbonized sorbents are summarized in Table 1.

INGO-2 sorbent has greater specific surface of 910 m²/g as compared with CBPS sorbent of 238 m²/g. Comparison of porosities of INGO-2 and CBPS sorbents shows that in CBPS sample porosity is 0.21 sm³/g, and in INGO-2 sample is 2.10 sm³/g. Absorption of methylene blue with carbonized INGO-2 sorbent is 270 mg/g and with carbonized CBPS sorbent is 234 mg/g.

**Table 1**

| Specific surface, porosity and adsorption activity of INGO-2 and CBPS sorbents | | |
|---|---|---|
| Parameter | Sorbent type | |
| | INGO-2 | CBPS |
| Porosity, cm³/g | 2,10 | 0,21 |
| Specific surface, m²/g | 910 | 238 |
| Absorption activity in re methylene blue, mg/g | 270 | 234 |

Thus, the porosity of INGO-2 sorbent increases in 10 times as compared with the porosity of CBPS sorbent, and the specific surface of INGO-2 sorbent increases more, than in 3.8 times as compared with CBPS sorbent, the absorption of methylene blue with carbonized sorbents is in 1.15 times more for INGO-2 than for CBPS sorbent.

### Example 3

Experiments of LPS adsorption in INGO-2 sorbent exhibite very high efficiency of enterosorbent for LPS binding (Figure).

LPS adsorption kinetics indicates that the high number of LPS in a buffer solution was adsorbed in CBPS for the first 40 minutes and in INGO-2 for the first 20 minutes. Obtained kinetics and adsorption data show the advantage of INGO-2 sorbent for selective sorption of enterotoxin, as LPS is completely adsorbed in the sorbent during the first 20 minutes of the adsorption process.

Thus, using the claimed method allows obtaining the product for enterosorption with higher sorption ability regarding LPS enterotoxin in comparison with CBPS.

### Example 4

Sorption activities of microbic cells of INGO-2 and CBPS enterosorbents are summarized in Table 2.

Studying microorganism sorption efficiency exhibites that INGO-2 enterosorbent has higher sorption characteristics concerning microbic cells, rather than CBPS. Further that is especially important; sorption capacity of cells of gram-negative bacterium is 10 times more, than gram-positive bacterium. The obtained information about sorption properties of rice husks ash in relation to gram-negative bacterium allows considering use thereof as perspective and very important, especially in the conditions of growth of levels of microbe resistance to antibacterial agents.

**Table 2**

| Sorption efficiency of gram-negative bacteria and gram-positive bacteria cells in INGO-2 and CBPS enterosorbents | | | |
|---|---|---|---|
| Test-organism strains | Cell initial concentration x 10⁹ | Cell sorption capacity (10⁸ per 1 g of sorbent) | |
| | | INGO-2 | CBPS |
| Gram-positive bacteria | | | |
| *L. plantarum AB-5* | 1.3±0.04 | 6.1±0.6 | 5.6±0.2 |
| *L. brevis AB-4* | 2.4±0.6 | 8.6±0.3 | 9.1±0.8 |
| *B. bifidum C-16* | 1.7±0.2 | 9.1±0.4 | 7.7±0.1 |
| *S. aureus S60* | 1.8±0.2 | 9.2±0.3 | 7.5±0.1 |

| Gram-negative bacteria | | | |
|---|---|---|---|
| *S. sonnei 158* | 2.5±0.4 | 62.1±0.9 | 8.5±0.4 |
| *S. typhimurium T1* | 2.0±0.3 | 73.4±0.5 | 9.4±0.6 |
| *E. cloacae 12* | 2.3±0.08 | 84.1±0.8 | 9.9±0.9 |

In this connection possibility of using this sorbent for microflora correction appears at the dysbacterioses connected with increase population level of gram-negative enterobacteriums.

### Example 5

Experiment data on determination of sorbents influence on bowels microflora of animals in experiments *in vivo* are summarized in Table 3. Sorbents are peroral administrated in dose of 25 mg/kg for 7 days.

As a result of a research of INGO-2 enterosorbent influence on animals it is found that INGO-2 enterosorbent does not have acute toxicity and chronic toxicity.

However for successful using enterosorbents it is necessary to define absence of negative influence thereof on normoflora. In this connection a research of INGO-2 enterosorbent influence on indicators of microbic structure of thick gut of experimental animals is conducted.

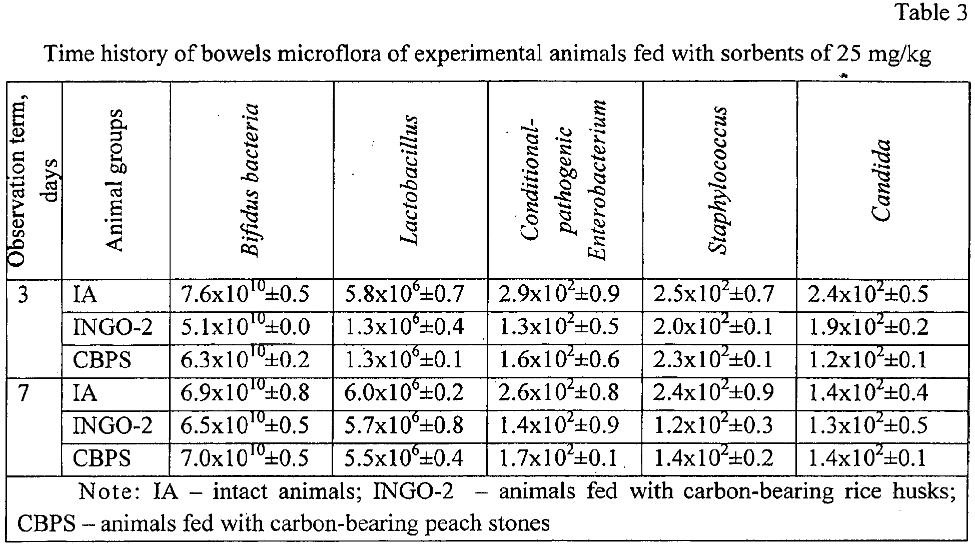

Laboratory animals are divided into 6 groups (10 units in each):
1 - intact animals;
2 - animals fed with INGO-2 enterosorbent in a dose of 25 mg/kg for 7 days;
3 - animals fed with CBPS in a dose of 25 mg/kg for 7 days.

Researches are carried out on white outbred rats that peroral administrated sorbents in doses of 25 mg/kg for 7 days. To define the maintenance of the basic groups of indigenius bacteria a sorbent administration following an inoculation of intestinal discharge of intact animals on differential diagnostic medium are made. Amounts of *Lactobacillus, Bifidus bacteria, Conditional-pathogenic enterobacterium,* staphylococcus, yeast fungus of *Candida genus* are identified. This data and also results obtained after definition of structure of intestinal microflora of rats fed with INGO-2 and CBPS in the specified doses on 3 and 7 days after the beginning of experiments are presented in Table 3.

### Example 6

Results of experiments of definition of sorbents influence on bowels microflora of animals in experiments *in vivo* are summarized in Table 4. Sorbents are peroral administrated in dose of 100 mg/kg for 7 days.

As a result of a research of INGO-2 enterosorbent influence on animals it is found that INGO-2 enterosorbent does not have acute toxicity and chronic toxicity.

However for successful using enterosorbents it is necessary to define absence of negative influence thereof on normoflora. In this connection a research of INGO-2 enterosorbent influence on indicators of microbic structure of thick gut of experimental animals is conducted.

Laboratory animals are divided into 6 groups (10 units in each):
1 - intact animals;
2 - animals fed with INGO-2 enterosorbent in a dose of 100 mg/kg for 7 days;
3 - animals fed with CBPS in a dose of 100 mg/kg for 7 days

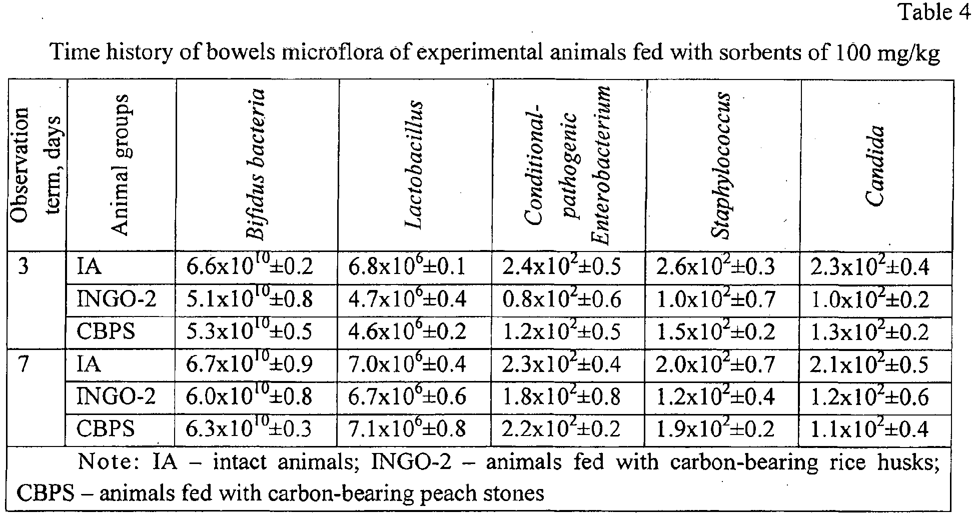

Researches are carried out on white outbred rats that peroral administrated sorbents in doses of 100 mg/kg for 7 days. To define the maintenance of the basic groups of indigenius bacteria a sorbent administration following an inoculation of intestinal discharge of intact animals on differential diagnostic medium are made. Amounts of *Lactobacillus, Bifidus bacteria, Conditional-pathogenic enterobacterium,* staphylococcus, yeast fungus of *Candida genus* are identified. This data and also results obtained after definition of structure of intestinal microflora of rats fed with INGO-2 and CBPS in the specified doses on 3 and 7 days after the beginning of experiments are presented in Table 4.

It is established, that on 7^{th} day after administration of INGO-2 in the used doses, good shows of this sorbent on qualitative and quantitative structure of thick intestines microflora of experimental animals are not marked (p > 0.05). And, decreasing titre of all analyzed groups of microorganisms in from 2 to 8 times is occurred though the first 3 days, but further investigated parameters come back to initial levels, i.e. there are not revealed statistical distinctions in the microbic passport of intestinal discharges of intact animals and skilled animals.

Thus, claimed INGO-2 enterosorbent provides more effective treatment-and-prophylactic effect on a human body or an animal body at acute poisoning and others endogenous intoxication and exogenous intoxication at the expense of higher colonization activity in bowels, and also INGO-2 enterosorbent has longer periods of storage.

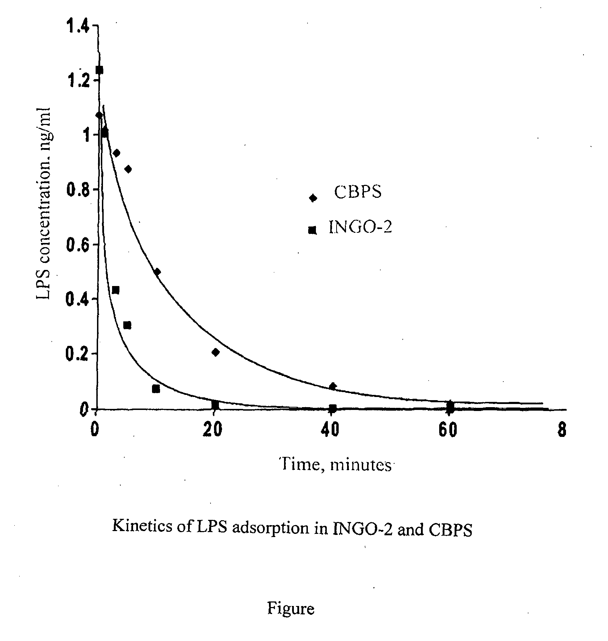

## Claims

1. Method for production of carbon enterosorbent, including a carbonization of a plant raw material followed by an isothermal ageing for a time ranging from 20 to 30 minutes, washing, drying, **characterized in that** rice husks are used as the plant raw material, the isothermal ageing is carried out at temperature of 650±10°C, and before washing the carbonized plant material is demineralized with 6M solution of hydrochloric acid at 100°C for a time ranging from 4 to 10 hours followed by neutralization with alkali.

## Patentansprüche

1. Verfahren zur Herstellung von Kohlenstoffenterosorbens, das die Carbonisierung eines pflanzlichen Rohstoffs und dessen anschliessende isothermische Alterung während eines Zeitraums von 20 bis 30 Minuten, Waschen und Trocknen umfasst,
**dadurch gekennzeichnet, dass** als pflanzlicher Rohstoff Reisschalen verwendet werden, die isothermische Alterung bei einer Temperatur von 650±10°C durchgeführt wird und das carbonisierte Pflanzenmaterial vor dem Waschen mit einer 6M-Salzsäurelösung bei 100°C während eines Zeitraums von 4 bis 10 Stunden demineralisiert und anschliessend mit Alkali neutralisiert wird.

## Revendications

1. Procédé pour la production d'entérosorbant carboné, y compris une carbonisation d'une matière première végétale suivit d'un vieillissement isothermique pour une durée de 20 à 30 minutes, lavage, séchage, **caractérisé en ce que** les envelopes de riz sont utilisées comme matière premiere végétale, le vieillisement isothermique est effectué à une température de 650±10°C, et avant le lavage la matière végétale carbonisée est déminéralisée avec une solution 6M d'acide hydrochlorique à 100°C pour une durée de 4 à 10 heures suivit par une neutralisation avec de l'alcali.
